# EUROPEAN PATENT SPECIFICATION

(11) **EP 3 565 621 B1**
(45) Date of publication and mention of the grant of the patent: **01.09.2021**
(21) Application number: 17701240.8
(22) Date of filing: 09.01.2017
(51) Int. Cl.: A61M 11/00, A61M 15/00, B05B 17/06, B05B 17/00

(54) **AEROSOL DELIVERY DEVICE AND METHOD FOR MANUFACTURING AND OPERATING THE SAME**
AEROSOLABGABEVORRICHTUNG UND VERFAHREN ZUR HERSTELLUNG UND ZUM BETRIEB DAVON
DISPOSITIF DE DISTRIBUTION D'AÉROSOL ET SON PROCÉDÉ DE FABRICATION ET D'UTILISATION

(43) Date of publication of application: 13.11.2019
(73) Proprietor: United Therapeutics Corporation, Silver Spring, MD 20910 (US)
(72) Inventor: BIETTE, Doug, Cary, North Carolina 27513 (US)
(74) Representative: Hoffmann Eitle
(86) International application number: PCT/US2017/012685
(87) International publication number: WO 2018/128629

(56) References cited:
- WO-A1-2007/028203
- DE-C- 962 296
- US-A- 2 863 075
- US-A- 3 360 664
- US-A- 3 561 444

## Description

### FIELD

The present disclosure relates generally to an aerosol delivery device and method for manufacturing and operating the same.

### BACKGROUND

Aerosol delivery devices such as nebulizers and inhalers (e.g., a metered dose inhaler) are useful for conveying an active substance in liquid form, together with respiratory air, into the lungs of a user. In particular, the aerosol delivery device transforms a predetermined concentration of an active substance into an aerosol via an atomizer. The aerosol is inhaled by the user into his or her lungs. From the lungs, the active substance is rapidly transferred to the blood stream with low loss. US 3,561,444 A discloses such aerosol delivery device and forms the basis for the preamble of claim 1, 2 and 8.

Variations in blood concentration of a drug can lead to inadequate efficacy if too little drug is present in the blood or at the site of action for any length of time, and to side effects when there is too much drug in the bloodstream or at the site of action. An ideal drug administration modality would achieve a steady concentration of the drug in the "therapeutic window" sufficient to achieve maximum efficacy, while not high enough to engender side effects.

The particle size of an inhaled formulation is an important consideration. Generally, the lungs are designed to exclude particles, so any inhaled formulation must overcome this barrier. The particle size can affect the amount of delivered particles and location of delivery. For example, particles that are too large may not reach deep into lung tissue, and particles that are too small may be exhaled before being deposited and absorbed. Thus, selecting an appropriate particle size and maintaining that particle size are important functions when developing an aerosol delivery device for a pharmaceutical product.

Because an exact dosage of the active substance delivered to the user may vary due to variations in the aerosol particle size, a need exists for improved technology, including a method for delivering a desired aerosol particle size using an aerosol delivery device, where the particle size is controlled, for example, to deliver an accurate dose of the active substance.

### SUMMARY

In one embodiment, a method of manufacturing an aerosol delivery device comprises the features of claim 1.

In another embodiment, a method of operating an aerosol delivery device comprises the features of claim 2.

In yet another embodiment, an adjustable aerosol delivery device comprises the features of claim 8.

Additional features, advantages, and aspects of the present disclosure may be set forth from consideration of the following detailed description, drawings, and claims.

### BRIEF DESCRIPTION OF THE DRAWINGS

The disclosure will become more fully understood from the following detailed description, taken in conjunction with the accompanying figures, in which:
FIG. 1 is a front view of a portion of an assembled aerosol delivery device including an active liquid container, a carrier liquid container, a piezo disc housing and an oscillator.
FIG. 2 is an exploded view of the portion of the aerosol delivery device of FIG. 1.
FIG. 3 is an cross-sectional view of a portion of the aerosol delivery device of FIG. 1.
FIG. 4A is a top, cross-sectional view of a bushing fixed relative to a carrier liquid container of the aerosol delivery device of FIG. 1.
FIG. 4B is a side view and a cross-sectional view of a fixing portion engaged with a fixing section of the bushing at a portion A of FIG. 4A.
FIG. 4C is a magnified cross-sectional view of a fixing portion engaged with a fixing section of the bushing at a portion A of FIG. 4A.

### DETAILED DESCRIPTION

Before turning to the figures, which illustrate the exemplary embodiments in detail, it should be understood that the present application is not limited to the details or methodology set forth in the description or illustrated in the figures. It should also be understood that the terminology is for the purpose of description only and should not be regarded as limiting.

### COMPONENTS OF THE AEROSOL DELIVERY DEVICE

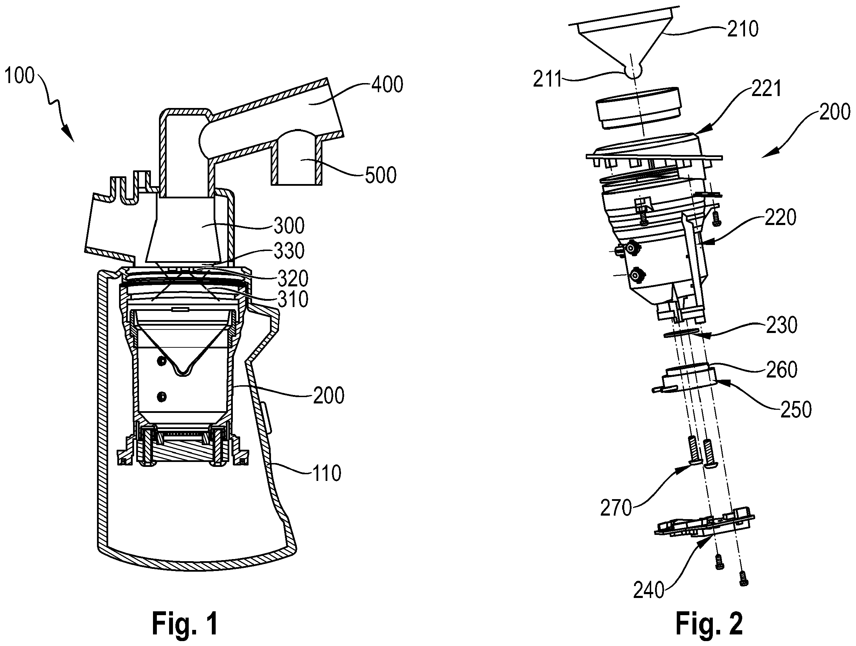

Referring to FIGS. 1 and 2, an adjustable aerosol delivery device 100, such as a nebulizer or an inhaler, is configured to deliver a liquid having an active substance dissolved therein in aerosol form to a user. The aerosol delivery device 100 also includes an atomizer 200 configured to transform the liquid having the active substance dissolved therein into an aerosol configured to be inhaled by a user. The active substance may be, for example, a medicine configured to be inhaled by a user in the form of an aerosol. The active substance may be used, for example, to treat respiratory conditions. In one embodiment, the active substance can be treprostinil or a pharmaceutically acceptable salt thereof. Treprostinil is approved for the treatment of pulmonary hypertension.

According to a preferred embodiment, the aerosol delivery device 100 may include a dome assembly 300 that connects a mouthpiece, an inhalation piece, and/or a mask 400 to the aerosol delivery device 100. That atomizer 200 (which will be described in detail below) transforms liquid having the active substance dissolved therein into an aerosol configured to be inhaled by a user. The aerosol flows upwards into the dome assembly 300. When a user inhales through the mouthpiece, inhalation piece and/or mask 400, the aerosol within the dome assembly 300 flows upwards and passes through slits 320 in a conical holder 330 of the dome assembly 300 and into the mouthpiece, inhalation piece and/or mask 400. Together, the slits 320 and the conical holder 330 comprise a baffle plate 310.

A particle size of an active substance delivered by the aerosol delivery device 100 may be altered by varying a size (i.e., a width) or number of baffle plates 310 disposed, transverse to the flow, in the interspace between the dome assembly 300 and the active liquid container 210 of the atomizer 200. Varying a width or number of slits 320 alters the size of a gap between the baffle plate 310 and the dome assembly 300 through which the aerosol particles must fit before delivery to the user. In other words, the baffle plates 310 may function similar to a filter.

The aerosol delivery device 100 may optionally include an exhalation port 500 connected to the dome assembly 300. Air exhaled by the user that enters the aerosol delivery device 100, for example, through the mouthpiece, inhalation piece and/or mask 400, may escape through the exhalation port 500. In particular, exhale air within the dome assembly 300 is diverted away from the dome and slits 320 via the exhalation port 500.

According to a preferred embodiment, the atomizer 200 includes an active liquid container 210 configured to receive and contain the liquid having the active substance dissolved therein. The active liquid container 210 may be, for example, a substantially conical active liquid container. The active liquid container 210 is preferably configured and formed of material(s) such that the active liquid container 210 will cause the liquid having the active substance dissolved therein to form an aerosol in response to oscillations generated by an oscillator 240 and transmitted by the carrier liquid, as described below.

According to a preferred embodiment, the atomizer 200 includes a carrier liquid container 220 configured to receive and contain a carrier liquid that can transmit oscillations. The carrier liquid preferably is water and, more preferably, is distilled water. The carrier liquid container 220 is configured to receive the active liquid container 210 in an inverted orientation such that only an apex 211 of the active liquid container 210 is submerged in the carrier liquid disposed in the carrier liquid container 220. The base of the active liquid container 210 is configured to rest, for example, on a flange 221 provided in an interior of the carrier liquid container 220. The atomizer 200 is configured to be received in a device chamber 110 of the aerosol delivery device 100. The device chamber 110 may be a hollow, center portion of the aerosol delivery device 100.

According to a preferred embodiment, the atomizer 200 includes a piezo disc 230. The piezo disc 230 is contained within a piezo disc housing 250 disposed between a base of the carrier liquid container 220 and the oscillator 240. The piezo disc 230 may be, for example, a ceramic disc or a metallic disc. The piezo disc 230 is configured to convert an electrical signal into a mechanical action (i.e., oscillation).

A flexible gasket 260 is provided at a top surface of the piezo disc housing 250 and preferably is configured to mount the piezo disc 230 relative to the carrier liquid container 220 and to seal a space between the mating surfaces of the carrier liquid container 220 and the piezo disc housing 250. As used in regard to the flexible gasket 260, the term flexible means that the gasket is elastic in that the gasket resume its normal shape after being stretched or compressed. In one example, the gasket may have a Shore A value of 35 to 55. In another example, the gasket may have a Shore A value of 40 to 50. In yet another example, the gasket may have an average Shore A value of 45.

The piezo disc housing 250 can be fixed in position relative to the carrier liquid container 220. Preferably, the piezo disc housing 250 is mounted to a base of the carrier liquid container 220 via a bushing 280 fixed within the carrier liquid container 220 and at least one fastener 270 extending through the piezo disc housing 250 and into the bushing 280. The bushing 280 is preferably configured to mate with the fastener 270, e.g., it may be threaded. According to a preferred embodiment, the fastener 270 is configured to be loosened or tightened to manipulate a compression of the flexible gasket 260 to adjust a particle size of the aerosol. By altering a gasket compression, it is possible to achieve particle size distribution equivalence, which allows for the maintenance of emitted dose equivalence. In one experiment, manipulating the gasket compression of the same aerosol delivery device allowed for an alteration in a particle size of the active substance delivered by the aerosol delivery device to be at least in a range of ± 0.5 microns.

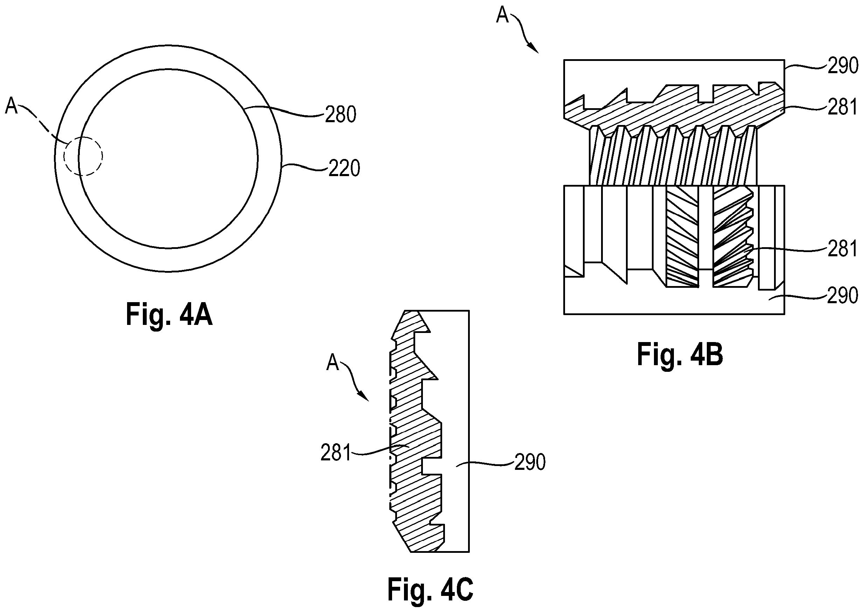

During operation of the aerosol delivery device 100, some bushings may expand when subjected to the vibrations of the oscillator 240 and/or an increase in temperature associated with the vibrations of the oscillator 240 and then fall back to a relaxed state, for example, when the aerosol delivery device 100 is no longer operated. To prevent the bushing 280 from becoming loose, the bushing 280 is rigidly fixed to the carrier liquid container 220. In particular, the bushing 280 is fixed relative to the carrier liquid container 220 via a fixing portion 290. The fixing portion 290 may be, for example, a knurl such as a straight tooth knurl, an angled knurl, a spiral knurl, a diamond knurl, etc. FIG. 4A illustrates a top, cross-sectional view of the bushing 280 fixed relative to the carrier liquid container 220. FIGS. 4B and 4C illustrate the fixing portion 290 engaging with a fixing section 281 of the bushing 280, for example, at a portion A, disposed at an interface between the carrier liquid container 220 and the bushing 280. The fixing portion 290 conforms to and mates with an entire perimeter of the fixing section 281 of the bushing 280. The fixing section 281 of the bushing 280 may be a partial section of an outer surface area of the bushing 280, an entire outer surface area of the bushing 280, or an entire outer surface area of the bushing 280 excluding a top face and a bottom face of the bushing 280.

In one example, the bushing 280 is insert molded in the fixing portion 290 such that the fixing portion 290 conforms to and mates with an entire perimeter of the fixing section 281 of the bushing 280. In the insert molding process, the bushing 280 is used as an insert placed in a mold, and material is injected into the mold around the bushing 280 and cooled to form the fixing portion 290 and at least a portion of the carrier liquid container 220.

In another example, an insert hole having a diameter larger than a diameter of the bushing 280 is formed at a base of the carrier liquid container 220 in a desired location of the bushing 280. The bushing 280 is inserted into the insert hole and molten plastic or adhesive (e.g., epoxy) is poured into the insert hole between the carrier liquid container 220 and an exterior of the bushing 280 (i.e., molten plastic or adhesive is poured around an exterior of the bushing 280). The plastic or adhesive cures within the insert hole to rigidly hold the bushing 280 in place, as the contours of the exterior of the bushing 280 are filled in with plastic or adhesive. The plastic or adhesive forms the fixing portion 290 that conforms to and mates with the entire perimeter of the fixing section 281 of the bushing 280.

In yet another example, the bushing 280 may be ultrasonically welded to the carrier liquid container 220. The weld forms the fixing portion 290 that conforms to and mates with the entire perimeter of the fixing section 281 of the bushing 280.

Fixing the bushing 280 to the carrier liquid container 220, as described in the examples above, results in the bushing 280 being more rigidly fixed to the carrier liquid container 220 than in a situation in which an insert hole having a diameter substantially the same as a diameter of the bushing is formed in a base of the carrier liquid container, and the bushing is inserted into the insert hole and held in place by friction fit and/or the force of the fastener received in the bushing. Fixing the bushing 280 to the carrier liquid container 220, as described in the examples above, also results in the bushing 280 being more rigidly fixed to the carrier liquid container 220 than in a situation in which an insert hole having a diameter larger than a diameter of the bushing is formed in a base of the carrier liquid container, and the bushing, having a flared design, is inserted into the insert hole and held in place by flared portion of the bushing. As a result, the loosening or relaxation of the bushing over time, which may affect particle size and the dosage of medication delivered by the aerosol device, can be prevented.

The bushing 280 may be selected from any known bushing including a solid sleeve bushing that is solid around a circumference of the bushing, a split bushing that has a cut along a length of the bushing, and clenched bearing that has a cut along a length of the bushing with a clench or cinch traversing the cut, provided that a pull force of the bushing 280 is greater than a counter force of the gasket 260. One of ordinary skill in the art would understand that the pull force of the bushing is the amount of force as measured, for example, by a pull force gauge, required to pull the bushing 280 out of the carrier liquid container 220. For example, the pull force of the bushing may be greater than or equal to 50 pounds. One of ordinary skill in the art would also understand that the counter force of the gasket 260 is a force generated when the gasket 260 is compressed, which is based on a durometer of the gasket 260.

A bearing surface of the bushing 280 may be threaded or smooth. In some embodiments, the bushing 280 may be formed of a rigid material, for example, metallic materials, such as at least one of brass, aluminum, copper, bronze, steel, or stainless steel. As used in regard to the bushing 280, the term rigid means that it has a stiffness, *k*, greater than or equal to a predetermined stiffness. For example, the predetermined bushing stiffness may be in the kN range. Stiffness, *k,* can be determined by the equation *k* = *F* / δ; where F is the force applied on the body; and δ is the displacement produced by the force along the same degree of freedom. In some embodiments, the bushing 280 may be a metallic bushing or a bi-metallic bushing. In other embodiments, the bushing 280 may be a plastic bushing. In additional embodiments, the bushing 280 may be formed of multiple materials, e.g., plastic encased within a metal shell. The bushing 280 may be formed of any material, as long as the pull force of the bushing 280 is greater than the counter force of the gasket 260. Although the bushing 280 is rigidly fixed to the carrier liquid container 220, one of ordinary skill in the art would understand that the mounting of the piezo disc housing 250 to the base of the carrier liquid container 220 is flexible and adjustable by changing the torque of the at least one fastener 270.

The fastener 270 is preferably configured to mate with the bushing 280, e.g., it may be threaded. The fastener 270 is preferably a bolt that can be threaded into the bushing 280. In some embodiments, the fastener 270 may be formed of a rigid material, for example, metallic materials, such as at least one of brass, aluminum, copper, bronze, steel, or stainless steel. As used in regard to the fastener 270, the term rigid means that it has a stiffness, *k*, greater than or equal to a predetermined stiffness. For example, the predetermined fastener stiffness may be in the kN range. Stiffness, *k,* can be determined by the equation *k* = *F* / δ; where *F* is the force applied on the body; and δ is the displacement produced by the force along the same degree of freedom. The fastener 270 may be formed of any material that provides the desired rigidity. For example, any material may be selected, so long as the material exceeds the pull force of the bushing 280 (e.g., the 50 pound force described above). Typically, metallic materials, such as at least one of brass, aluminum, copper, bronze, steel, or stainless steel, will provide the desired rigidity. For example, the fastener 270 may be a M12 stainless steel screw with tension/shear capacities in the kN range. The fastener 270 may be formed of multiple materials, e.g., plastic encased within a metal shell, as long as it provides the desired rigidity at the area of contact with the bushing 280.

In one embodiment, the aerosol delivery device 100 includes an oscillator 240 that is configured to generate an ultrasonic wave that causes the piezo disc 230 to oscillate and transmit the oscillations through the carrier liquid to the active liquid to cause at least a portion of the active liquid to become an aerosol. The oscillator 240 can be configured to have a desired frequency, which preferably is at least or nominally 2.4 MHz. The oscillator 240 can have a single oscillating element or can be comprised of a plurality of oscillating elements, having a net frequency that equals the desired frequency, e.g., at least or nominally 2.4 MHz. The mechanical vibration of the piezo disc 230 generates a mist on the surface of the liquid in the active liquid container 220, which separates the liquid in the active liquid container 210 into an aerosol, which is a mixture of air and particles of the active substance. The particle size of the active substance is very small. For example, when the active substance is treprostinil, a distribution of the particle size is centered on 2 microns. In some embodiments, the particle size may be 4 microns or less, in particular, 2 to 4 microns.

### METHODS FOR ALTERING A PARTICLE SIZE OF THE ACTIVE SUBSTANCE DELIVERED BY THE AEROSOL DELIVERY DEVICE

It may be desirable to manufacture an aerosol delivery device that can be manipulated to deliver the same active substance with different particle sizes.

In one embodiment (described in detail below), a particle size of the same active substance delivered by the same aerosol delivery device 100 is altered by manipulating a mean compression of the gasket 260. Increasing the mean compression of the gasket 260 allows the atomizer 200 to produce smaller particle sizes of the same active substance, while decreasing the compression of the gasket 260 allows the atomizer 200 to produce larger particle sizes of the same active substance. In some instances, it is preferable to produce smaller particle sizes of the active substance. The mean compression of the gasket 260 may be altered, for example, by loosening or tightening the at least one fastener 270 that mounts the piezo disc housing 250 to the base of the carrier liquid container 220.

The bushing 280 of the aerosol delivery device 100 remains rigid whether or not the aerosol delivery device 100 is operating. This rigidity allows for a more accurate compression of the gasket 260. Although the bushing 280 is rigid, one of ordinary skill in the art would understand that the mounting of the piezo disc housing 250 to the base of the carrier liquid container 220 is flexible and adjustable by changing the torque of the at least one fastener 270. By changing the torque of the at least one fastener 270, a pressure exerted on the gasket 260 may be finely controlled. The mean compression of the gasket 260 may be altered, for example, in a range from 0.1 to 1.0 mm, in particular, from 0.5 to 0.7 mm, and even more particularly from 0.51 to 0.66 mm.

It is believed that altering the mean compression of the gasket 260 alters translation of an energy focal point when comparing aerosol delivery devices having the same torque specifications of the fastener 270. Altering the mean compression of the gasket 260 is believed to alter the proportion of energy directed into the liquid of the carrier liquid container 220 versus the energy directed into the piezo disc housing 250 (including the piezo disc 230 and the gasket 260). The fastener 270 may be loosened or tightened to achieve a desired amount of rigidity between the carrier liquid container 220 and the piezo disc housing 250 via the bushing 280 in which the fastener 270 is mounted. It is preferable to tighten the fastener 270, but leave the fastener 270 loose enough such that a predetermined minimum proportion of the energy is delivered to the piezo disc housing 250 and absorbed by the gasket 260. If the fastener 270 is over-tightened (i.e., over-torqued), the connection of the piezo disc housing 250 to the base of the carrier liquid container 220 may become rigid, and all of the energy may be delivered to the liquid of the carrier liquid container 220, thereby causing the piezo disc 230 to crack if ceramic. If the piezo disc 230 is metallic, the piezo disc 230 may fail due to loss of piezo material on the disc. Altering the mean compression of the gasket 260 may also alter the resonant frequency of the piezo disc 230. In addition, because the gasket 260 constrains motion at the edge of the piezo disc 230, altering the mean compression of the gasket 260 may also change the mechanical load. For at least these reasons, altering the mean compression of the gasket 260 by loosening or tightening the fastener 270 will allow for a change in particle size of the active substance delivered from the mask or the mouthpiece of the aerosol delivery device 100.

Other alternatives may be used to adjust the particle size of the active substance delivered by the aerosol delivery device 100, provided that a net actuation frequency of the oscillator 240 is at least 2.4 MHz and the mounting of the piezo disc housing 250 to the base of the active liquid container 220 is flexible.

As one alternative, the carrier liquid container 220 itself may be threaded, either as part of the molding process or by using plastic cutting screws. In this embodiment, the bushing 280 is eliminated such that the fastener 270 is directly inserted into an insert hole formed in a base of the carrier liquid container 220.

As another alternative, the material of the gasket 260 may be changed to one having a stiffer or a softer durometer. Even if the same force is applied by the fastener 270, using a gasket 260 having a different material would change the compression force.

### MANUFACTURING THE AEROSOL DELIVERY DEVICE

A method for manufacturing an aerosol delivery device 100 includes disposing the atomizer 200 and the dome assembly 300 at least partially within the device chamber 110 and inserting the mouthpiece, inhalation piece and/or mask 400 into the dome assembly 300. The method includes disposing the active liquid container 210 within the carrier liquid container 220, providing the piezo disc 230 within the piezo disc housing 250, mounting the piezo disc housing 250 to a base of the carrier liquid container 220, sealing a space between the mating surfaces of the carrier liquid container 220 and the piezo disc housing 250 with a gasket 260, and providing an oscillator 240 at a base of the piezo disc housing 250. The oscillator 240 is configured to generate an ultrasonic wave that causes the piezo disc 230 to vibrate and separate the liquid in the active liquid container 210 into an aerosol containing air and particles of the active substance. The method of manufacturing the aerosol delivery device 100 further includes manipulating a compression of the gasket 260 to alter a particle size of the active substance that the aerosol delivery device 100 is configured to produce. Increasing the compression of the gasket reduces the particle size of the active substance that the aerosol delivery device is configured to produce.

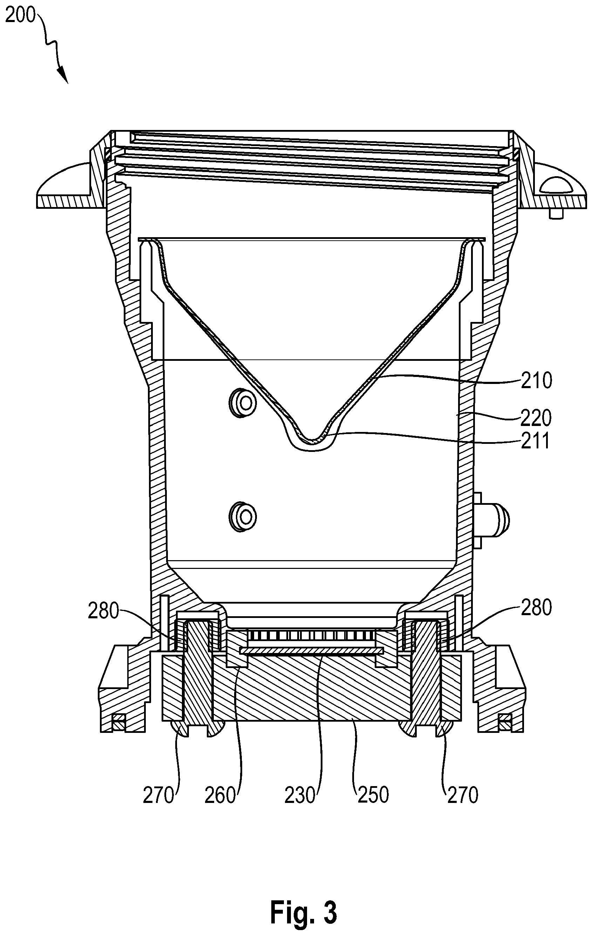

As seen in FIG. 3, in one embodiment, the piezo disc housing 250 is mounted to the base of the carrier liquid container 220 using at least one fastener 270 that is received in a bushing 280 rigidly fixed to the carrier liquid container 220, for example, by insert molding. The bushing 280 may be selected from any known bushing including a solid sleeve bushing that is solid around a circumference of the bushing, a split bushing that has a cut along a length of the bushing, and clenched bearing that has a cut along a length of the bushing with a clench or cinch traversing the cut. The bushing 280 may be a metallic bushing, a bi-metallic bushing, or a plastic bushing. In the embodiments in which the bushing 280 is a metallic bushing or a bi-metallic bushing, the bushing 280 may be made, for example, from brass, aluminum, copper, bronze, steel, or stainless steel. In at least one embodiment, the bushing 280 is not a plastic bushing.

The method of manufacturing the aerosol delivery device 100 may further include altering at least one of an orientation of the piezo disc 230 within the piezo disc housing 250 and an orientation of the gasket 260 within the piezo disc housing 250 to further alter the particle size of the active substance that the aerosol delivery device 100 is configured to produce.

The method of manufacturing the aerosol delivery device 100 may further include altering a calibration of the aerosol delivery device 100 to further alter the particle size of the active substance that the aerosol delivery device 100 is configured to produce. Altering a calibration of the aerosol delivery device 100 may be done as an alternative to, or in addition to altering at least one of an orientation of the piezo disc 230 within the piezo disc housing 250 and an orientation of the gasket 260 within the piezo disc housing 250 to further alter the particle size of the active substance that the aerosol delivery device 100 is configured to produce.

### OPERATING THE AEROSOL DELIVERY DEVICE

A method of operating the aerosol delivery device 100 includes introducing the carrier liquid into the carrier liquid container 220, introducing the liquid having the active substance dissolved therein into the active liquid container 210, inserting the active liquid container 210 into the carrier liquid container 220 such that a portion of the active liquid container 210 is submerged in the carrier liquid, generating an ultrasonic wave with the oscillator 240 that causes the piezo disc 230 to oscillate and transmit the oscillations through the carrier liquid to the active liquid to cause at least a portion of the active liquid to become an aerosol, and manipulating a compression of the gasket 260 to alter a particle size of the active substance produced by the aerosol delivery device 100.

The method of operating the aerosol delivery device 100 further includes increasing the compression of the gasket 260 to reduce the particle size of the active substance produced by the aerosol delivery device 100. The compression of the gasket 260 may be manipulated by loosening or tightening the at least one fastener 270 used to mount the piezo disc housing 250 to the base of the carrier liquid container 220.

The method of operating the aerosol delivery device 100 may further include altering at least one of an orientation of the piezo disc 230 within the piezo disc housing 250 and an orientation of the gasket 260 within the piezo disc housing 250 to further alter the particle size of the active substance produced by the aerosol delivery device 100.

The method of operating the aerosol delivery device 100 may further include altering a calibration of the aerosol delivery device 100 to further alter the particle size of the active substance produced by the aerosol delivery device 100. Altering the calibration of the aerosol delivery device 100 may be done as an alternative to or in addition to altering at least one of an orientation of the piezo disc 230 within the piezo disc housing 250 and an orientation of the gasket 260 within the piezo disc housing 250 to further alter the particle size of the active substance produced by the aerosol delivery device 100.

As utilized herein, the terms "approximately," "about," "substantially", and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described and claimed without restricting the scope of these features to the precise numerical ranges provided. Accordingly, these terms should be interpreted as indicating that insubstantial or inconsequential modifications or alterations of the subject matter described and claimed are considered to be within the scope of the invention as recited in the appended claims.

The terms "coupled," "connected," and the like as used herein mean the joining of two members directly or indirectly to one another. Such joining may be stationary (e.g., permanent) or moveable (e.g., removable or releasable). Such joining may be achieved with the two members or the two members and any additional intermediate members being integrally formed as a single unitary body with one another or with the two members or the two members and any additional intermediate members being attached to one another.

References herein to the positions of elements (e.g., "top," "bottom," "above," "below," etc.) are merely used to describe the orientation of various elements in the FIGURES. It should be noted that the orientation of various elements may differ according to other exemplary aspects, and that such variations are intended to be encompassed by the present disclosure.

It is important to note that the construction and arrangement of the aerosol delivery device as shown in the various exemplary aspects are illustrative only. Although only a few aspects have been described in detail in this disclosure, those skilled in the art who review this disclosure will readily appreciate that many modifications are possible (e.g., variations in sizes, dimensions, structures, shapes and proportions of the various elements, values of parameters, mounting arrangements, use of materials, colors, orientations, etc.) without materially departing from the novel teachings and advantages of the subject matter described herein. For example, elements shown as integrally formed may be constructed of multiple parts or elements, the position of elements may be reversed or otherwise varied, and the nature or number of discrete elements or positions may be altered or varied. The order or sequence of any process or method steps may be varied or re-sequenced according to alternative aspects. Other substitutions, modifications, changes and omissions may also be made in the design, operating conditions and arrangement of the various exemplary aspects without departing from the scope of the present invention as defined in the appended claims.

## Claims

1. A method of manufacturing an aerosol delivery device (100) comprising:
disposing an active liquid container within a carrier liquid container, wherein the active liquid container (210) is configured to contain an active liquid having an active substance dissolved therein and the carrier liquid container (220) is configured to contain a carrier liquid; and
mounting a piezo disc (230) with a flexible gasket (260) relative to the carrier liquid container (220), wherein the piezo disc (230) is configured to be oscillated by ultrasonic waves generated by an oscillator (240) and transmit the oscillations through the carrier liquid to the active liquid to cause at least a portion of the active liquid to become an aerosol; **characterized in that**
mounting the piezo disc (230) relative to the active liquid container includes inserting at least one fastener (270) through a piezo disc housing (250) holding the piezo disc (230) and into a bushing (280) fixed within the carrier liquid container (220); and by
manipulating a compression of the gasket (260) by loosening or tightening the at least one fastener (270) to adjust a particle size of the aerosol to be produced.

2. A method of operating an aerosol delivery device (100) including an active liquid container (210) configured to contain an active liquid having an active substance dissolved therein, a carrier liquid container (220) configured to receive a carrier liquid, a piezo disc (230) mounted with a flexible gasket (260) relative to the carrier liquid container (220), wherein the piezo disc (230) is configured to be oscillated by ultrasonic waves generated by an oscillator (240) and transmit the oscillations through the carrier liquid to the active liquid to cause at least a portion of the active liquid to become an aerosol, the method comprising:
introducing the carrier liquid into the carrier liquid container (220);
introducing the active liquid having the active substance dissolved therein into the active liquid container (210);
inserting the active liquid container (210) into the carrier liquid container (220) such that a portion of the active liquid container (210) is submerged in the carrier liquid; and
generating an ultrasonic wave with the oscillator (240) that causes the piezo disc (230) to oscillate and transmit the oscillations through the carrier liquid to the active liquid to cause at least a portion of the active liquid to become an aerosol; **characterized in** by
mounting the piezo disc (230) relative to the active liquid container includes inserting at least one fastener (270) through a piezo disc housing (250) holding the piezo disc (230) and into a bushing (280) fixed within the carrier liquid container (220); and by
manipulating a compression of the gasket (260) by loosening or tightening at least one fastener (270) to alter a particle size of aerosol.

3. The method of claim 1 or 2, wherein manipulating the compression of the flexible gasket (260) includes increasing the compression of the flexible gasket (260) to reduce the particle size of the aerosol or decreasing the compression of the flexible gasket (260) to increase the particle size of the aerosol.

4. The method of claim 1 or 2, wherein a pull force of the bushing (280) is greater than a counter force of the gasket (260).

5. The method of claim 4, wherein fixing the bushing (280) relative to the carrier liquid container comprises providing a fixing portion (290) that conforms to and mates with an entire perimeter of a fixing section (281) of the bushing (280) to fix the bushing (280) relative to the carrier liquid container (220).

6. The method of claim 5, wherein fixing the bushing (280) relative to the carrier liquid container (220) comprises insert molding the fixing portion (290) such that the fixing portion (290) conforms to and mates with the entire perimeter of the fixing section (281) of the bushing (280).

7. The method of claim 5, wherein fixing the bushing (280) relative to the carrier liquid container comprises:
providing an insert hole in a base of the carrier liquid container (220), the insert hole having a diameter greater than a diameter of the bushing (280);
inserting the bushing (280) into the insert hole; and
introducing adhesive material into the insert hole between an exterior surface of the bushing (280) and the carrier liquid container (220), the adhesive material conforming to and mating with the entire perimeter of the fixing section (281) of the bushing (280).

8. An adjustable aerosol delivery device (100), comprising:
an active liquid container (210) configured to contain an active liquid having an active substance dissolved therein;
a carrier liquid container (220) configured to receive a carrier liquid;
a piezo disc (230)
a flexible gasket (260);
at least one fastener (270); and
an oscillator (240) configured to generate an ultrasonic wave that causes the piezo disc (230) to oscillate and transmit the oscillations through the carrier liquid to the active liquid to cause at least a portion of the active liquid to become an aerosol; **characterized in that**
the piezo disc (230) is disposed in and held by a piezo disc housing (250) wherein the piezo disc housing (250) is disposed between a base of the carrier liquid container (220) and the oscillator (240);
the flexible gasket (260) is configured to mount the piezo disc (230) relative to the carrier liquid container (220),
a bushing (280) is fixed relative to the carrier liquid container (220) via a fixing portion (290), the fixing portion (290) conforming to and mating with an entire perimeter of a fixing section (281) of the bushing (280); and
the at least one fastener (270) extends through the piezo disc housing (250) and into the bushing (280),
wherein the at least one fastener (270) is configured to be loosened or tightened to manipulate a compression of the gasket (260) to adjust a particle size of the aerosol.

9. The adjustable aerosol delivery device of claim 8, wherein the bushing (280) is insert molded in the fixing portion (290) such that the fixing portion (290) conforms to and mates with the entire perimeter of the fixing section (281) of the bushing (280).

10. The adjustable aerosol delivery device of claim 8, wherein the fixing portion (290) includes adhesive material that conforms to and mates with the entire perimeter of the fixing section (281) of the bushing (280).

11. The adjustable aerosol delivery device of claim 8, wherein the fixing portion (290) includes plastic material that conforms to and mates with the entire perimeter of the fixing section (281) of the bushing (280).

12. The adjustable aerosol delivery device of claim 8, wherein the at least one fastener (270) is configured increase the compression of the flexible gasket (260) to reduce the particle size of the aerosol or decrease the compression of the flexible gasket (260) to increase the particle size of the aerosol.

13. The adjustable aerosol delivery device of claim 8, wherein a pull force of the bushing (280) is greater than a counter force of the gasket (260).

## Patentansprüche

1. Verfahren zum Herstellen einer Aerosolabgabevorrichtung (100), das umfasst:
Anordnen eines Aktivflüssigkeitsbehälters innerhalb eines Trägerflüssigkeitsbehälters, wobei der Aktivflüssigkeitsbehälter (210) konfiguriert ist, um eine aktive Flüssigkeit zu enthalten, die eine aktive Substanz aufweist, die darin aufgelöst ist, und der Trägerflüssigkeitsbehälter (220) konfiguriert ist, um eine Trägerflüssigkeit zu enthalten; und
Montieren einer Piezoscheibe (230) mit einer flexiblen Dichtung (260) in Bezug zu dem Trägerflüssigkeitsbehälter (220), wobei die Piezoscheibe (230) konfiguriert ist, um von Ultraschallwellen, die von einem Oszillator (240) erzeugt werden, oszilliert zu werden und die Oszillationen durch die Trägerflüssigkeit zu der aktiven Flüssigkeit zu übertragen, um mindestens einen Abschnitt der aktiven Flüssigkeit zu veranlassen, ein Aerosol zu werden; **dadurch gekennzeichnet, dass**
das Montieren der Piezoscheibe (230) in Bezug zu dem Aktivflüssigkeitsbehälter das Einsetzen mindestens einer Befestigung (270) durch ein die Piezoscheibe (230) haltendes Piezoscheibengehäuse (250) und in eine in dem Trägerflüssigkeitsbehälter (220) befestigte Durchführung (280) beinhaltet; und durch
Handhaben einer Kompression der Dichtung (260) durch Lockern oder Anziehen der mindestens einen Befestigung (270), um eine Teilchengröße des zu erzeugenden Aerosols einzustellen.

2. Verfahren zum Betreiben einer Aerosolabgabevorrichtung (100), die einen Aktivflüssigkeitsbehälter (210) beinhaltet, der konfiguriert ist, um eine aktive Flüssigkeit zu enthalten, die eine aktive Substanz aufweist, die darin aufgelöst ist, einen Trägerflüssigkeitsbehälter (220), der konfiguriert ist, um eine Trägerflüssigkeit aufzunehmen, eine Piezoscheibe (230), die mit einer flexiblen Dichtung (260) in Bezug zu dem Trägerflüssigkeitsbehälter (220) montiert ist, wobei die Piezoscheibe (230) konfiguriert ist, um von Ultraschallwellen, die von einem Oszillator (240) erzeugt werden, oszilliert zu werden, und die Oszillationen durch die Trägerflüssigkeit zu der aktiven Flüssigkeit zu übertragen, um mindestens einen Abschnitt der aktiven Flüssigkeit zu veranlassen, ein Aerosol zu werden, wobei das Verfahren umfasst:
Einführen der Trägerflüssigkeit in den Trägerflüssigkeitsbehälter (220);
Einführen der aktiven Flüssigkeit, die eine aktive Substanz aufweist, die darin aufgelöst ist, in den Aktivflüssigkeitsbehälter (210);
Einsetzen des Aktivflüssigkeitsbehälters (210) in den Trägerflüssigkeitsbehälter (220) derart, dass ein Abschnitt des Aktivflüssigkeitsbehälters (210) in der Trägerflüssigkeit eingetaucht ist; und
Erzeugen eine Ultraschallwelle mit dem Oszillator (240), die die Piezoscheibe (230) veranlasst zu oszillieren, und die Oszillationen durch die Trägerflüssigkeit zu der aktiven Flüssigkeit zu übertragen, um mindestens einen Abschnitt der aktiven Flüssigkeit zu veranlassen, ein Aerosol zu werden; **gekennzeichnet durch**
das Montieren der Piezoscheibe (230) in Bezug zu dem Aktivflüssigkeitsbehälter das Einsetzen mindestens einer Befestigung (270) durch ein die Piezoscheibe (230) haltendes Piezoscheibengehäuse (250) und in eine in dem Trägerflüssigkeitsbehälter (220) befestigte Durchführung (280) beinhaltet; und durch
Handhaben einer Kompression der Dichtung (260) durch Lockern oder Anziehen mindestens einer Befestigung (270), um eine Teilchengröße des Aerosols zu verändern.

3. Verfahren nach Anspruch 1 oder 2, wobei das Handhaben der Kompression der flexiblen Dichtung (260) das Erhöhen der Kompression der flexiblen Dichtung (260) beinhaltet, um die Teilchengröße des Aerosols zu reduzieren, oder die Kompression der flexiblen Dichtung (260) zu verringern, um die Teilchengröße des Aerosols zu erhöhen.

4. Verfahren nach Anspruch 1 oder 2, wobei eine Zugkraft der Durchführung (280) größer ist als eine Gegenkraft der Dichtung (260).

5. Verfahren nach Anspruch 4, wobei das Befestigen der Durchführung (280) in Bezug zu dem Trägerflüssigkeitsbehälter das Bereitstellen eines Befestigungsabschnitts (290) umfasst, der mit einem gesamten Umfang eines Befestigungsteilabschnitts (281) der Durchführung (280) übereinstimmt und zusammenpasst, um die Durchführung (280) in Bezug zu dem Trägerflüssigkeitsbehälter (220) zu befestigen.

6. Verfahren nach Anspruch 5, wobei das Befestigen der Durchführung (280) in Bezug zu dem Trägerflüssigkeitsbehälter (220) das Umspritzen des Befestigungsabschnitts (290) derart umfasst, dass der Befestigungsabschnitt (290) mit dem gesamten Umfang des Befestigungsteilabschnitts (281) der Durchführung (280) übereinstimmt und zusammenpasst.

7. Verfahren nach Anspruch 5, wobei das Befestigen der Durchführung (280) in Bezug zu dem Trägerflüssigkeitsbehälter umfasst:
Bereitstellen einer Einsetzbohrung in einer Basis des Trägerflüssigkeitsbehälters (220), wobei die Einsetzbohrung einen Durchmesser aufweist, der größer ist als ein Durchmesser der Durchführung (280).
Einsetzen der Durchführung (280) in die Einsetzbohrung; und
Einführen von Klebematerial in die Einsetzbohrung zwischen einer Außenoberfläche der Durchführung (280) und dem Trägerflüssigkeitsbehälter (220), wobei das Klebematerial mit dem gesamten Umfang des Befestigungsteilabschnitts (281) der Durchführung (280) übereinstimmt und zusammenpasst.

8. Einstellbare Aerosolabgabevorrichtung (100), die umfasst:
einen Aktivflüssigkeitsbehälter (210), der konfiguriert ist, um eine aktive Flüssigkeit zu enthalten, die eine aktive Substanz aufweist, die darin aufgelöst ist;
einen Trägerflüssigkeitsbehälter (220), der konfiguriert ist, um eine Trägerflüssigkeit aufzunehmen;
eine Piezoscheibe (230);
eine flexible Dichtung (260);
mindestens eine Befestigung (270); und
einen Oszillator (240), der konfiguriert ist, um eine Ultraschallwelle zu erzeugen, die die Piezoscheibe (230) veranlasst, zu oszillieren, und die Oszillationen durch die Trägerflüssigkeit zu der aktiven Flüssigkeit zu übertragen, um mindestens einen Abschnitt der aktiven Flüssigkeit zu veranlassen, ein Aerosol zu werden; **dadurch gekennzeichnet, dass**
die Piezoscheibe (230) in einem Piezoscheibengehäuse (250) angeordnet ist und davon gehalten wird, wobei die Piezoscheibengehäuse (250) zwischen einer Basis des Trägerflüssigkeitsbehälters (220) und dem Oszillator (240) angeordnet ist;
eine flexible Dichtung (260) konfiguriert ist, um die Piezoscheibe (230) in Bezug zu dem Trägerflüssigkeitsbehälter (220) zu montieren,
eine Durchführung (280) in Bezug zu dem Trägerflüssigkeitsbehälter (220) über einen Befestigungsabschnitt (290) befestigt ist, wobei der Befestigungsabschnitt (290) mit einem gesamten Umfang eines Befestigungsteilabschnitts (281) der Durchführung (280) übereinstimmt und zusammenpasst; und
sich die mindestens eine Befestigung (270) durch das Piezoscheibengehäuse (250) und in die Durchführung (280) erstreckt,
wobei die mindestens eine Befestigung (270) konfiguriert ist, um gelockert oder angezogen zu werden, um eine Kompression der Dichtung (260) zu handhaben, um eine Teilchengröße des Aerosols einzustellen.

9. Einstellbare Aerosolabgabevorrichtung nach Anspruch 8, wobei die Durchführung (280) derart in dem Befestigungsabschnitt (290) umspritzt ist, dass der Befestigungsabschnitt (290) mit dem gesamten Umfang des Befestigungsteilabschnitts (281) der Durchführung (280) übereinstimmt und zusammenpasst.

10. Einstellbare Aerosolabgabevorrichtung nach Anspruch 8, wobei der Befestigungsabschnitt (290) Klebematerial beinhaltet, das mit dem gesamten Umfang des Befestigungsteilabschnitts (281) der Durchführung (280) übereinstimmt und zusammenpasst.

11. Einstellbare Aerosolabgabevorrichtung nach Anspruch 8, wobei der Befestigungsabschnitt (290) Kunststoffmaterial beinhaltet, das mit dem gesamten Umfang des Befestigungsteilabschnitts (281) der Durchführung (280) übereinstimmt und zusammenpasst.

12. Einstellbare Aerosolabgabevorrichtung nach Anspruch 8, wobei die mindestens eine Befestigung (270) konfiguriert ist, um die Kompression der flexiblen Dichtung (260) zu erhöhen, um die Teilchengröße des Aerosols zu reduzieren, oder die Kompression der flexiblen Dichtung (260) zu verringern, um die Teilchengröße des Aerosols zu erhöhen.

13. Einstellbare Aerosolabgabevorrichtung nach Anspruch 8, wobei eine Zugkraft der Durchführung (280) größer ist als eine Gegenkraft der Dichtung (260).

## Revendications

1. Procédé de fabrication d'un dispositif de distribution d'aérosol (100) comprenant :
la disposition d'un récipient de liquide actif à l'intérieur d'un récipient de liquide porteur, dans lequel le récipient de liquide actif (210) est configuré pour contenir un liquide actif présentant une substance active dissoute dans celui-ci et le récipient de liquide porteur (220) est configuré pour contenir un liquide porteur ; et
le montage d'un disque piézoélectrique (230) avec un joint d'étanchéité flexible (260) par rapport au récipient de liquide porteur (220), dans lequel le disque piézoélectrique (230) est configuré pour osciller sous l'effet d'ondes ultrasonores générées par un oscillateur (240) et transmettre les oscillations à travers le liquide porteur vers le liquide actif pour amener au moins une portion du liquide actif à se transformer en aérosol ; **caractérisé en ce que**
le montage du disque piézoélectrique (230) par rapport au récipient de liquide actif inclut l'insertion d'au moins un élément de fixation (270) à travers un logement de disque piézoélectrique (250) maintenant le disque piézoélectrique (230) et dans une douille (280) fixée à l'intérieur du récipient de liquide porteur (220) ; et par
la manipulation d'une compression du joint d'étanchéité (260) par desserrage ou serrage de l'au moins un élément de fixation (270) pour ajuster une taille de particule de l'aérosol à produire.

2. Procédé de fonctionnement d'un dispositif de distribution d'aérosol (100) incluant un récipient de liquide actif (210) configuré pour contenir un liquide actif présentant une substance active dissoute dans celui-ci, un récipient de liquide porteur (220) configuré pour recevoir un liquide porteur, un disque piézoélectrique (230) monté avec un joint d'étanchéité flexible (260) par rapport au récipient de liquide porteur (220), dans lequel le disque piézoélectrique (230) est configuré pour pouvoir osciller sous l'effet d'ondes ultrasonores générées par un oscillateur (240) et transmettre les oscillations à travers le liquide porteur vers le liquide actif pour amener au moins une portion du liquide actif à se transformer en aérosol, le procédé comprenant :
l'introduction du liquide porteur dans le récipient de liquide porteur (220) ;
l'introduction du liquide actif présentant la substance active dissoute dans celui-ci dans le récipient de liquide actif (210) ;
l'insertion du récipient de liquide actif (210) dans le récipient de liquide porteur (220) de sorte qu'une portion du récipient de liquide actif (210) soit immergée dans le liquide porteur ; et
la génération d'une onde ultrasonore avec l'oscillateur (240) pour amener le disque piézoélectrique (230) à osciller et transmettre les oscillations à travers le liquide porteur vers le liquide actif pour amener au moins une portion du liquide actif à se transformer en aérosol ; **caractérisé par**
le montage du disque piézoélectrique (230) par rapport au récipient de liquide actif inclut l'insertion d'au moins un élément de fixation (270) à travers un logement de disque piézoélectrique (250) maintenant le disque piézoélectrique (230) et dans une douille (280) fixée à l'intérieur du récipient de liquide porteur (220) ; et par
la manipulation d'une compression du joint d'étanchéité (260) par desserrage ou serrage d'au moins un élément de fixation (270) pour altérer une taille de particule d'aérosol.

3. Procédé selon la revendication 1 ou 2, dans lequel la manipulation de la compression du joint d'étanchéité flexible (260) inclut l'augmentation de la compression du joint d'étanchéité flexible (260) pour réduire la taille de particule de l'aérosol ou la diminution de la compression du joint d'étanchéité flexible (260) pour augmenter la taille de particule de l'aérosol.

4. Procédé selon la revendication 1 ou 2, dans lequel une force de traction de la douille (280) est supérieure à une force contraire du joint d'étanchéité (260).

5. Procédé selon la revendication 4, dans lequel la fixation de la douille (280) par rapport au récipient de liquide porteur comprend la fourniture d'une portion de fixation (290) qui se conforme et correspond à un périmètre total d'une section de fixation (281) de la douille (280) pour fixer la douille (280) par rapport au récipient de liquide porteur (220).

6. Procédé selon la revendication 5, dans lequel la fixation de la douille (280) par rapport au récipient de liquide porteur (220) comprend le moulage par insertion de la portion de fixation (290) de sorte que la portion de fixation (290) se conforme et corresponde au périmètre total de la section de fixation (281) de la douille (280).

7. Procédé selon la revendication 5, dans lequel la fixation de la douille (280) par rapport au récipient de liquide porteur comprend :
la fourniture d'un trou d'insertion dans une base du récipient de liquide porteur (220), le trou d'insertion présentant un diamètre supérieur à un diamètre de la douille (280) ;
l'insertion de la douille (280) dans le trou d'insertion ; et
l'introduction d'une matière adhésive dans le trou d'insertion entre une surface extérieure de la douille (280) et le récipient de liquide porteur (220), la matière adhésive se conformant et correspondant au périmètre total de la section de fixation (281) de la douille (280).

8. Dispositif de distribution d'aérosol ajustable (100), comprenant :
un récipient de liquide actif (210) configuré pour contenir un liquide actif présentant une substance active dissoute dans celui-ci ;
un récipient de liquide porteur (220) configuré pour recevoir un liquide porteur ;
un disque piézoélectrique (230)
un joint d'étanchéité flexible (260) ;
au moins un élément de fixation (270) ; et
un oscillateur (240) configuré pour générer une onde ultrasonore qui amène le disque piézoélectrique (230) à osciller et transmettre les oscillations à travers le liquide porteur vers le liquide actif pour amener au moins une portion du liquide actif à se transformer en aérosol ; **caractérisé en ce que**
le disque piézoélectrique (230) est disposé dans et maintenu par un logement de disque piézoélectrique (250) dans lequel le logement de disque piézoélectrique (250) est disposé entre une base du récipient de liquide porteur (220) et l'oscillateur (240) ;
le joint d'étanchéité flexible (260) est configuré pour monter le disque piézoélectrique (230) par rapport au récipient de liquide porteur (220),
une douille (280) est fixée par rapport au récipient de liquide porteur (220) par l'intermédiaire d'une portion de fixation (290), la portion de fixation (290) se conformant et correspondant au périmètre total d'une section de fixation (281) de la douille (280) ; et
l'au moins un élément de fixation (270) s'étend à travers le logement de disque piézoélectrique (250) et dans la douille (280),
dans lequel l'au moins un élément de fixation (270) est configuré pour être desserré ou serré pour manipuler une compression du joint d'étanchéité (260) pour ajuster une taille de particule de l'aérosol.

9. Dispositif de distribution d'aérosol ajustable selon la revendication 8, dans lequel la douille (280) est moulée par insertion dans la portion de fixation (290) de sorte que la portion de fixation (290) se conforme et corresponde au périmètre total de la section de fixation (281) de la douille (280).

10. Dispositif de distribution d'aérosol ajustable selon la revendication 8, dans lequel la portion de fixation (290) inclut une matière adhésive qui se conforme et correspond au périmètre total de la section de fixation (281) de la douille (280).

11. Dispositif de distribution d'aérosol ajustable selon la revendication 8, dans lequel la portion de fixation (290) inclut une matière plastique qui se conforme et correspond au périmètre total de la section de fixation (281) de la douille (280).

12. Dispositif de distribution d'aérosol ajustable selon la revendication 8, dans lequel l'au moins un élément de fixation (270) est configuré pour augmenter la compression du joint d'étanchéité flexible (260) pour réduire la taille de particule de l'aérosol ou diminuer la compression du joint d'étanchéité flexible (260) pour augmenter la taille de particule de l'aérosol.

13. Dispositif de distribution d'aérosol ajustable selon la revendication 8, dans lequel une force de traction de la douille (280) est supérieure à une force contraire du joint d'étanchéité (260).
